Europäisches Patentamt

European Patent Office

Office européen des brevets

(11) Veröffentlichungsnummer: **0 170 784**
A1

## (12) EUROPÄISCHE PATENTANMELDUNG

(21) Anmeldenummer: **85105134.2**

(22) Anmeldetag: **26.04.85**

(51) Int. Cl.⁴: **A 61 M 5/14**

(30) Priorität: **05.06.84 DE 3420865**

(43) Veröffentlichungstag der Anmeldung: **12.02.86**
**Patentblatt 86/7**

(84) Benannte Vertragsstaaten: **AT BE CH DE FR GB IT LI LU NL SE**

(71) Anmelder: **Intermedicat GmbH, Gerliswilstrasse 45, CH-6020 Emmenbrücke (CH)**

(72) Erfinder: **Köhler, Uwe, Elisabethweg 12, D-3588 Homberg (DE)**
Erfinder: **Rosskopf, Gerhard, Heiligenstrasse 29, D-3501 Dörnhagen (DE)**

(74) Vertreter: **von Kreisler, Alek, Dipl.-Chem. et al, Deichmannhaus am Hauptbahnhof, D-5000 Köln 1 (DE)**

(54) **Infusionsspritzenpumpe.**

㊾ Die Infusionsspritzenpumpe weist ein aufklappbares Gehäuse (10) auf, in dem eine Faltenbalgspritze (18) untergebracht ist. Die Antriebsvorrichtung (39), die sich ebenfalls im Gehäuse (10) befindet, treibt einen Halter (33), der am rückwärtigen Ende der Faltenbalgspritze (18) befestigt ist, in Richtung auf einen den Hals (17) der Spritze fixierenden weiteren Halter (16). Die Infusionsspritzenpumpe ist vollständig gekapselt. Sie hat geringe Abmessungen und kann als tragbares Gerät, das am Körper des Patienten mitgeführt wird, ausgebildet sein.

ACTORUM AG

Infusionsspritzenpumpe

Die Erfindung betrifft eine Infusionsspritzenpumpe mit einem ersten Halter zum Befestigen des Halses einer Spritze, einem relativ zu dem ersten Halter linear bewegbaren zweiten Halter zum Ausdrücken der Spritze und mit einer den zweiten Halter antreibenden Antriebsvorrichtung.

Derartige Infusionsspritzenpumpen dienen dazu, einem Patienten ein flüssiges Medikament kontinuierlich und in genau bemessener Dosierung zuzuführen. Die bekannten Spritzenpumpen sind für Kolbenspritzen bestimmt. Kolbenspritzen haben im gefüllten Zustand etwa die doppelte Länge des das Medikament aufnehmenden Spritzenzylinders. Die Länge der Pumpe entspricht der maximalen Länge der Spritze. Dies führt dazu, daß die Pumpen große Abmessungen haben und schwergewichtig sind. Die Spritzen werden bei den bekannten Infusionsspritzenpumpen am Gehäuse außen an zwei vom Gehäuse abstehenden Haltern befestigt.

Bekannt sind ferner Infusionsspritzen, die als Balgspritzen ausgebildet sind (US-PS 4 411 656, EP 0 094 101). Balgspritzen haben im gefüllten Zustand eine geringere Länge als Kolbenspritzen, weil keine abstehende Kolbenstange vorhanden ist.

Schließlich ist eine oszillierende Verdrängerpumpe bekannt (DE-PS 28 23 802), bei der ein ziehharmonikaartiger Balg, der an jedem Ende mit einem Rückschlagventil versehen ist, von einem Linearantrieb abwechselnd zusammengedrückt und auseinandergezogen wird, um aus einer Einlaßleitung Flüssigkeit anzusaugen und diese in eine Auslaßleitung zu pumpen. Der Balg ist hierbei außerhalb des den Antrieb enthaltenden Gehäuses zwischen zwei Haltern angebracht.

Der Erfindung liegt die Aufgabe zugrunde, eine Infusionsspritzenpumpe der eingangs genannten Art zu schaffen, die geringere Abmessungen und ein geringeres Gewicht hat als die bekannten Infusionsspritzenpumpen und die leicht zu handhaben und zu bewegen ist.

Die Lösung dieser Aufgabe besteht erfindungsgemäß darin, daß die Spritze eine kolbenlose Faltenbalgspritze ist und daß der erste Halter Bestandteil eines Gehäuses ist, das die Faltenbalgspritze umschließt und in dem der zweite Halter bewegbar ist.

Die erfindungsgemäße Infusionsspritzenpumpe hat wegen der Verwendung einer Faltenbalgspritze eine Länge, die nur etwa der halben Länge der bekannten Infusionsspritzenpumpen entspricht. Dadurch ist es möglich, ein Gehäuse vorzusehen, das alle Teile der Pumpe, einschließlich der Spritze selbst, umschließt und das relativ kleinformatig gestaltet werden kann. Eine derartige Infusionsspritzenpumpe kann als mobiles Gerät ausgebildet sein, das am Körper eines Patienten mitgeführt wird. Der Patient braucht also nicht an eine stationäre Infusionsspritzenpumpe angeschlossen zu werden. In dem Gehäuse ist die Faltenbalgspritze geschützt untergebracht, so daß durch Anstoßen an externe Gegenstände keine Verformung der Spritze auftritt. Dies ist gerade bei einer Faltenbalgspritze wichtig, weil bei einer Deformierung des Faltenbalges, die nicht durch den zweiten Halter hervorgerufen ist, ein Medikamentenstoß in den Körper des Patienten gelangen würde. Durch das Gehäuse werden derartige Fehldosierungen vermieden.

Gemäß einer bevorzugten Ausführungsform der Erfindung weist das Gehäuse ein die Antriebsvorrichtung enthaltendes Unterteil und einen aufklappbaren Deckel auf. Der erste Halter kann aus zwei auseinanderklappbaren Gehäuseteilen gebildet sein, die Aussparungen aufweisen, welche sich im Schließzustand des Gehäuses zu einer den Hals der Spritze fixierenden Öffnung ergänzen. Nach dem Öffnen des Deckels ist die Faltenbalgspritze zugänglich, so daß das Auswechseln der Spritze sehr einfach auch von geübten Personen, z.B. vom Patienten selbst, durchgeführt werden kann.

Die üblichen Faltenbalgspritzen haben am vorderen Ende einen konischen Ansatz, durch den hindurch das Medikament ausgestoßen wird. Wenn die Rückwand der Spritze ebenflächig ist, ergibt sich durch den konischen Ansatz und durch die schrägverlaufende Spritzenschulter ein großer Totraum im Innern der Spritze, wenn diese im vollständig zusammengedrückten Zustand ist. Damit verbleibt ein erhebliches Restvolumen des Medikamentes in der Spritze. Um dieses Restvolumen zu reduzieren ist gemäß einer bevorzugten Weiterbildung der Erfindung vorgesehen, daß die Rückwand der Faltenbalgspritze eine in das Spritzeninnere hineinragende Ausformung aufweist, die im wesentlichen an die Innenkontur der Spritzenschulter angepaßt ist.

Der zweite Halter kann einen drehbaren Gewindestopfen aufweisen, der in ein Gewinde der Ausformung der Rückwand der Spritze einschraubbar ist. Auf diese Weise wird eine kraftschlüssige Verbindung zwischen dem bewegbaren zweiten Halter und dem rückwärtigen Teil der Spritze hergestellt. Der zweite Halter ist durch die Gewindeverbindung mit der Spritze verbunden, so daß er sich nicht in eine falsche Position verschieben kann.

Die Faltenbalgspritze kann entweder von Anfang an das Medikament enthalten und somit als Einwegspritze ausgebildet sein, die nach dem Entleeren fortgeworfen wird, es ist aber auch möglich, eine Faltenbalgspritze zu verwenden, die nach dem Entleeren erneut gefüllt werden kann. Hierzu ist es besonders zweckmäßig, wenn die Rückwand der Spritze durch kraftschlüssige Verbindung mit dem zweiten Halter zurückgezogen werden kann, um das Medikament in die Spritze einzusaugen. Vorzugsweise ist im Hals der Faltenbalgspritze eine durchstechbare Membran oder ein von einem externen Rohrstück zu öffnendes Ventil angebracht.

Im folgenden wird unter Bezugnahme auf die Zeichnungen ein Ausführungsbeispiel der Erfindung näher erläutert.

Es zeigen:

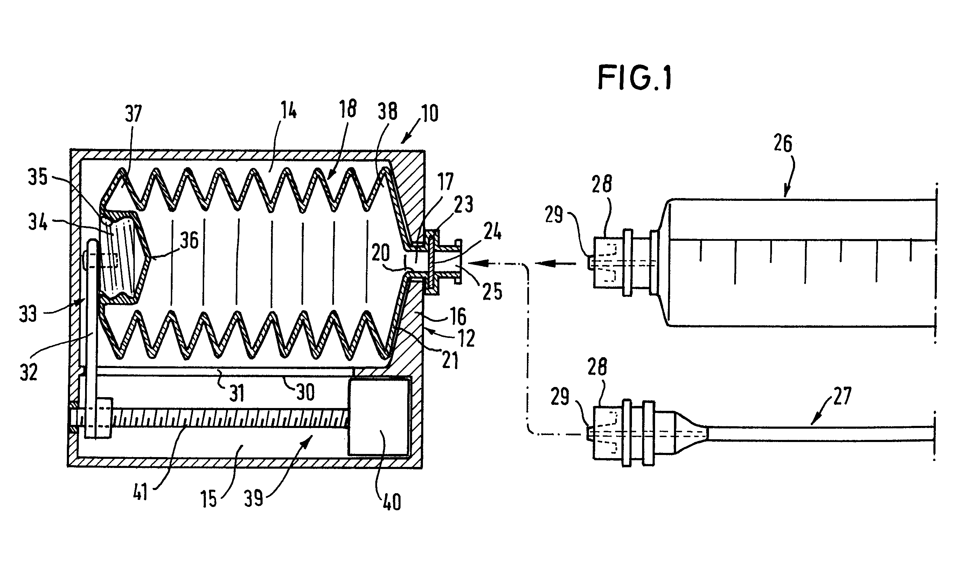

Fig. 1     einen horizontalen Längsschnitt durch die Infusionsspritzenpumpe und

Fig. 2     eine perspektivische Darstellung der
           Spritzenpumpe bei geöffnetem Gehäuse.


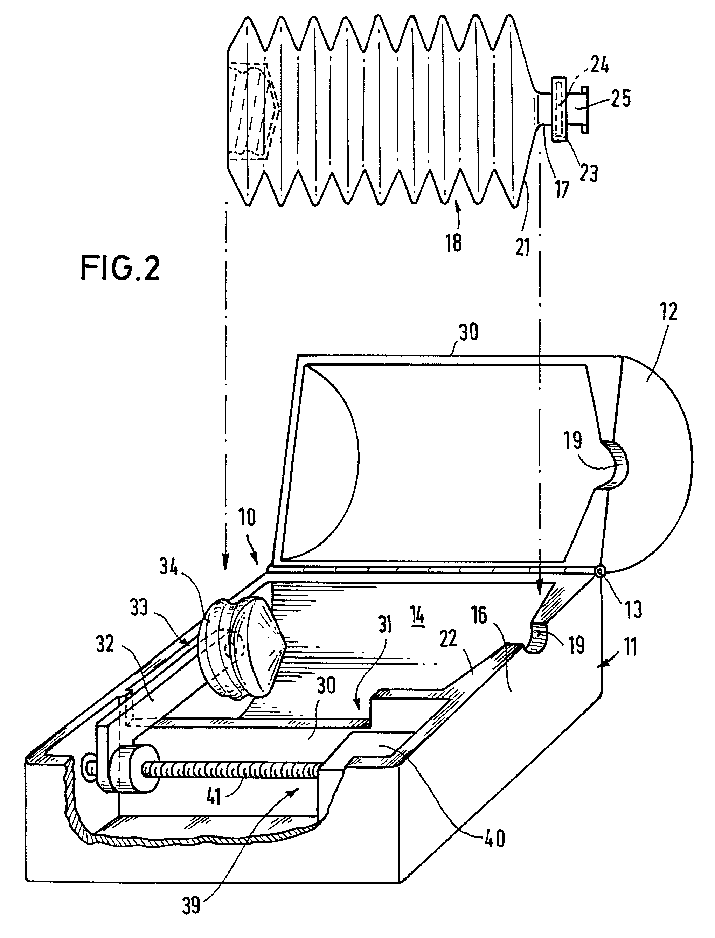

Die Infusionsspritzenpumpe weist ein zweiteiliges Gehäuse 10 auf, das aus einem Bodenteil 11 und einem Deckel 12 besteht. Der Deckel 12 ist an einer Längsseite des oberen Randes des Bodenteiles 11 mit einem Scharnier 13 befestigt, so daß er in der in Fig. 2 dargestellten Weise aufgeklappt werden kann. Im Gehäuse 10 befinden sich eine Spritzenkammer 14 und an der dem Scharnier 13 abgewandten Seite der Spritzenkammer eine Antriebskammer 15. Die Spritzenkammer 14 und die Antriebskammer 15 erstrecken sich jeweils über die gesamte Länge des Gehäuses 10.

Die vordere Stirnwand des Gehäuses 10 bildet den ersten Halter 16 zur Fixierung des Halses 17 der Faltenbalgspritze 18. Zu diesem Zweck sind in den beiden Teilen der Stirnwand halbkreisförmige Aussparungen 19 vorgesehen, die sich bei geschlossenem Deckel 12 zu einer kreisförmigen Öffnung 20 ergänzen, durch die der Hals 17 hindurchführt. Die kegelstumpfförmige Spritzenschulter 21 stützt sich an der kegelstumpfförmigen Innenseite 22 des ersten Halters 16 ab.

Der Hals 17 der Faltenbalgspritze 18 ist mit einem Ringflansch 23 versehen, welcher nicht durch die Öffnung 20 hindurchpaßt und an der den ersten Halter 16 bildenden Stirnwand des Gehäuses von außen anliegt, um zu verhindern, daß der Hals 17 in das Innere des Gehäuses 10 hineingedrückt werden kann. Das Innere des rohrförmigen Halses 17 ist durch eine weich-elastische geschlitzte Scheibe 24 verschlossen, um das Innere der Faltenbalgspritze 18 gegen das Eindringen von Kontaminationen zu schützen. Das frei abstehende äußere Ende des Halses 17

- 6 -

0170784

ist als Kupplungsstück 25 ausgebildet, an das entweder das hierzu passende Kupplungsstück 28 eines Injektionsbehälters 26 oder das Kupplungsstück 28 einer Schlauchleitung 27 angeschlossen werden kann. Das Kupplungsstück 28 enthält ein vorstehendes Rohrstück 29, das beim Ankuppeln an das Kupplungsstück 25 den Schlitz der Scheibe 24 durchdringt und somit den Behälter 26 bzw. den Schlauch 27 mit dem Innenraum der Faltenbalgspritze 18 verbindet.

Die Wand 30, die die Antriebskammer 15 von der Spritzenkammer 14 trennt, weist einen Längsschlitz 31 auf, durch den eine Stange 32 des zweiten Halters 33 quer hindurchgeht. An dem spritzenseitigen Ende der Stange 32 ist ein Gewindestopfen 34 drehbar gelagert. Dieser Gewindestopfen ist mit einem Außengewinde versehen, das in ein Innengewinde 35 der Faltenbalgspritze 18 eingeschraubt werden kann. Das Innengewinde 35 befindet sich in der Wand einer Ausformung 36, die von der Rückwand der Faltenbalgspritze in den Innenraum dieser Spritze hinein vorsteht. Wenn die Faltenbalgspritze vollständig zusammengedrückt ist, legt sich die Ausformung 36 gegen die Spritzenschulter 21, so daß das in der Spritze verbleibende Restvolumen gering gehalten wird. Die Spritze sollte blasenfrei gefüllt sein. Die Wandung der Faltenbalgspritze mit dem gewellten Mantel und der konisch zulaufenden Spritzenschulter ist geeignet, durch Senkrechthalten des Behältnisses eventuell nach der Füllung vorhandene Luftblasen durch die Ausflußöffnung austreten zu lassen, ggf. unter geringem Zusammenpressen des Faltenbalges von der Rückseite (mit Totraumverdränger) her.

Die in der Antriebskammer 15 enthaltene Antriebsvorrichtung 39 besteht bei dem vorliegenden Ausführungsbeispiel aus einem Motor 40, welcher eine Spindel 41 treibt, die mit der Stange 32 in Gewindeeingriff steht. Wenn der Motor die Schraubenspindel 41 treibt, wird die Stange 32 in Längsrichtung des Gehäuses 10 bewegt. Diese lineare Querbewegung der Stange 32 wird über den Gewindestopfen 34 auf die Rückwand der Faltenbalgspritze 18 übertragen, so daß diese Rückwand sich kontinuierlich dem vorderen Ende der Spritze nähert.

Wie aus Fig. 2 ersichtlich ist, hat die Spritzenkammer 14 eine im wesentlichen zylindrische Form, wobei die eine Hälfte des Zylinders von dem Unterteil 11 und die andere Hälfte von dem Deckel 12 des Gehäuses 10 gebildet wird.

Das Material der Faltenbalgspritze ist ein flexibler Kunststoff aus einem Material, das von dem Medikament nicht angegriffen wird und keine Substanzen oder Partikel in die Lösung freisetzt. Das Material der Faltenbalgspritze ist vorzugsweise lichtundurchlässig, um auch lichtempfindliche Medikamente mit der Spritze verabreichen zu können. Mindestens eine Wand der Spritzenkammer 14 des Gehäuses 10 ist durchsichtig, damit von außen festgestellt werden kann, wie weit die Spritze ausgepreßt ist, ohne das Gehäuse öffnen zu müssen.

ANSPRÜCHE:

1. Infusionsspritzenpumpe mit einem ersten Halter (16) zum Befestigen des Halses (17) einer Spritze, einem relativ zu dem ersten Halter (16) linear bewegbaren zweiten Halter (33) zum Ausdrücken der Spritze und mit einer den zweiten Halter (33) antreibenden Antriebsvorrichtung (39), d a d u r c h   g e k e n n z e i c h n e t , daß die Spritze eine kolbenlose Faltenbalgspritze (18) ist und daß der erste Halter (16) Bestandteil eines Gehäuses (10) ist, das die Faltenbalgspritze (18) umschließt und in dem der zweite Halter (33) bewegbar ist.

2. Infusionsspritzenpumpe nach Anspruch 1, dadurch gekennzeichnet, daß das Gehäuse (10) ein die Antriebsvorrichtung (39) enthaltendes Unterteil (11) und einen aufklappbaren Deckel (12) aufweist.

3. Infusionsspritzenpumpe nach Anspruch 1 oder 2, dadurch gekennzeichnet, daß der erste Halter (16) aus zwei auseinanderklappbaren Gehäuseteilen gebildet ist, die Aussparungen (19) aufweisen, welche sich im Schließzustand des Gehäuses zu einer den Hals (17) der Spritze (18) fixierenden Öffnung (20) ergänzen.

4. Infusionsspritzenpumpe nach einem der Ansprüche 1 bis 3, dadurch gekennzeichnet, daß die Rückwand der Faltenbalgspritze (18) eine in das Spritzeninnere hineinragende Ausformung (36) aufweist, die zur Verringerung des Restvolumens der Spritze im wesentlichen an die Innenkontur der Spritzenschulter (21) angepaßt ist.

5. Infusionsspritzenpumpe nach einem der Ansprüche 1 bis 4, dadurch gekennzeichnet, daß der zweite Halter (33) einen drehbaren Gewindestopfen (34) aufweist, der in ein Gewinde (35) einer Ausformung (36) der Rückwand der Spritze einschraubbar ist.

6. Infusionsspritzenpumpe nach einem der Ansprüche 1 bis 5, dadurch gekennzeichnet, daß das Gehäuse (10) in eine Spritzenkammer (14) und eine Antriebskammer (15) unterteilt ist und daß die diese Kammern trennende Wand (30) einen Längsschlitz (31) aufweist, durch den der zweite Halter (33) hindurchragt.

7. Infusionsspritzenpumpe nach einem der Ansprüche 1 bis 6, dadurch gekennzeichnet, daß im Hals (17) der Faltenbalgspritze (18) eine durchstechbare Membran oder ein von einem externen Rohrstück (29) zu öffnendes Ventil (24) angebracht ist.

# FIG.1

0170784

0170784

FIG.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | GB-A-2 072 017 (J.L. PETERS) <br> * Seite 1, Zeilen 44-88; Figuren 1, 2 * | 1,3,6 | A 61 M 5/14 |
| A | US-A-3 884 228 (S. HAHN) <br> * Spalte 2, Zeile 67 - Spalte 5, Zeile 22; Figuren 1, 2 * | 1,7 | |
| A | DE-A-2 945 405 (MINNESOTA MINING AND MANUFACTURING CO.) <br> * Ansprüche 1, 4; Figuren 1-5 * | 1-3 | |
| A | EP-A-0 016 343 (INTERMEDICAT GMBH) <br> * Ansprüche 1, 3; Figuren 1, 2 * | 1,6 | |
| A | DE-A-1 491 754 (W.R. JEWETT) <br> * Seite 4, Zeile 1 - Seite 5, Zeile 15; Figuren 1, 2 * | 1 | RECHERCHIERTE SACHGEBIETE (Int. Cl.4) <br><br> A 61 M 3/00 <br> A 61 M 5/00 |
| A | US-A-4 150 672 (D.G. WHITNEY et al.) <br> * Spalte 5, Zeile 4 - Spalte 7, Zeile 5; Figuren 1-6 * | 1,2,6,7 | |
| A | DE-A-1 566 635 (NOVAMPOULE AG) <br> * Anspruch 4; Figur 2 * | 4 | |
|  | --- -/- | | |

Der vorliegende Recherchenbericht wurde für alle Patentansprüche erstellt.

| Recherchenort <br> BERLIN | Abschlußdatum der Recherche <br> 23-08-1985 | Prüfer <br> MASSALSKI W. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

**Europäisches Patentamt**

**EUROPÄISCHER RECHERCHENBERICHT**

0170784

Nummer der Anmeldung

EP 85 10 5134

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-U-6 902 557 (WESTFORM PLASTIKWERKE GMBH) <br> * Seite 3, Zeilen 21,22; Figur * <br><br> ----- | 4 | |
| | | | **RECHERCHIERTE SACHGEBIETE (Int. Cl.4)** |

Der vorliegende Recherchenbericht wurde für alle Patentansprüche erstellt.

| Recherchenort <br> BERLIN | Abschlußdatum der Recherche <br> 23-08-1985 | Prüfer <br> MASSALSKI W. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82